# EUROPEAN PATENT APPLICATION

(11) **EP 3 437 556 A1**
(43) Date of publication of application: **06.02.2019**
(21) Application number: 16901584.9
(22) Date of filing: 13.05.2016
(51) Int. Cl.: A61B 5/11

(54) **BIOLOGICAL INFORMATION ACQUISITION DEVICE**

(71) Applicant: Daikin Industries, Ltd., Osaka-shi, Osaka 530-8323 (JP)
(72) Inventor: HIEI, Takehiko, Osaka-shi, Osaka 530-8323 (JP); TSUBOI, Chiaki, Osaka-shi, Osaka 530-8323 (JP); TORAMOTO, Sayo, Osaka-shi, Osaka 530-8323 (JP); SHIGEMORI, Kazuhisa, Osaka-shi, Osaka 530-8323 (JP)
(74) Representative: Goddar, Heinz J.
(86) International application number: PCT/JP2016/002347
(87) International publication number: WO 2017/195235

(57) **Abstract**

A seat portion (22) includes a first layer (61), a second layer (62), and a third layer (63) that are stacked sequentially from the surface side to the inner side of the seat portion (22). The second layer (62) has a higher stiffness than the third layer (63), and the first layer (61) has a lower stiffness than the third layer (63). A pressure sensitive part (31) has a pressure sensitive-part body (34) arranged between the first layer (61) and the second layer (62).

## Description

### TECHNICAL FIELD

The present invention relates to a biological information acquisition device.

### BACKGROUND ART

Conventionally, a biological information acquisition device that acquires biological information of a human body has been known.

For example, the biological information acquisition device described in Patent Document 1 is provided with a plurality of pressure sensitive parts (air bags) arranged in a backrest of an automotive seat. When a motion of a seated user is transmitted to the pressure sensitive parts, the biological information acquisition device acquires time series signal data including the pulse waves of aortae in the vicinity of the lumbar area of the seated user in association with air pressure fluctuations in the pressure sensitive part. The time series signal data is subjected to signal processing so that a hypnagogic sign is detected as biological information.

Patent Document 2 discloses a biological information acquisition device in which a pressure sensitive part (silicon tube) is buried in the internal intermediate portion of polyurethane foam that constitutes a mat (see FIG. 3 of Patent Document 2). When a motion of the body of a person lying sprawled on the mat is transmitted to the pressure sensitive part, the pressure change in the pressure sensitive part is detected by a pressure sensor. In the biological information acquisition device, biological information, such as cardiac beats and breathing, is acquired based on the signal detected by the pressure sensor.

### CITATION LIST

### PATENT DOCUMENT

PATENT DOCUMENT 1: Japanese Unexamined Patent Publication No. 2010-46236
PATENT DOCUMENT 2: Japanese Unexamined Patent Publication No. 2003-235828

### SUMMARY OF THE INVENTION

### TECHNICAL PROBLEM

Meanwhile, it is conceivable to arrange the above-mentioned pressure sensitive part in a seating article, such as a sofa, to obtain biological information of a human body (seated user). However, if the pressure sensitive part is simply arranged in the seat portion of the sofa, the pressure sensitive part becomes obstructive to the seated user, or the pressure sensitive part is brought into direct contact with the buttocks of the seated user, thus impairing the comfort of the seated user.

On the other hand, as described in Patent Document 2, it is also conceivable to bury the pressure sensitive part in the inside of a flexible member (polyurethane foam or the like) is also conceivable. However, if the pressure sensitive part is buried in this manner, the polyurethane foam considerably deforms together with the pressure sensitive part when a user is seated and hence, the following problems occur: that is, when the user is seated, a reduced change is caused in internal pressure of the pressure sensitive part, and the detection accuracy of the biological information is lowered.

The present invention has been made under such circumstances, and it is an object of the present invention to propose a biological information acquisition device capable of maintaining the comfort of the seated user, and detecting the biological information with high accuracy.

### SOLUTION TO THE PROBLEM

A first aspect of the present invention is directed to a biological information acquisition device including a seating article (21) having a backrest (23) and a seat portion (22); a pressure sensitive part (31) arranged in the seat portion (22), and configured to receive pressure caused by a motion of a seated user (S); and an information acquisition part (30) configured to acquire biological information of the seated user (S) based on the pressure received by the pressure sensitive part (31). The seat portion (22) includes a first layer (61), a second layer (62), and a third layer (63) that are stacked sequentially from a surface side to an inner side of the seat portion (22), and is configured such that the second layer (62) has a higher stiffness than the third layer (63), and the first layer (61) has a lower stiffness than the third layer (63). The pressure sensitive part (31) has a pressure sensitive-part body (34) arranged between the first layer (61) and the second layer (62).

In the first aspect, the motion of the user (S) seated on the seating article (21) acts on the pressure sensitive part (31) arranged in the seat portion (22). The information acquisition part (30) acquires the biological information of the seated user (S) based on the pressure caused by the motion of the seated user (S) and received by the pressure sensitive part (31).

The seat portion (22) of the seating article (21) includes the first layer (61), the second layer (62), and the third layer (63) that are stacked sequentially from the surface side to the inner side of the seat portion (22). The pressure sensitive-part body (34) of the pressure sensitive part (31) is arranged between the first layer (61) and the second layer (62). As a result, the pressure sensitive part (31) is covered with the first layer (61) that is more flexible than the second layer (62) and the third layer (63). Thus, the seated user (S) sits on the first layer (61) having flexibility, and the pressure sensitive part (31) is less likely to be perceived by the buttocks or the thighs of the seated user (S).

The second layer (62) has a higher stiffness than each of the first layer (61) and the third layer (63). That is, the second layer (62) having a comparatively high stiffness is arranged on the lower side of the pressure sensitive-part body (34). This feature can prevent the second layer (62) from being considerably deformed due to the motion of the seated user (S), enabling the pressure sensitive-part body (34) to reliably receive the pressure caused by the motion of the seated user (S). Thus, the detection accuracy of the pressure sensitive part (31) is improved.

The third layer (63) is more flexible than the second layer (62), ensuring the cushion performance of the seating article (21). This feature can ensure the comfort of the seated user (S).

A second aspect of the present invention is an embodiment of the first aspect. In the second aspect, the pressure sensitive part includes a pressure sensitive tube (31) whose internal pressure changes in response to the motion of the seated user (S), and the pressure sensitive-part body includes a tube body (34) arranged between the first layer (61) and the second layer (62).

In the second aspect, the tube body (34) of the pressure sensitive tube (31) is arranged between the first layer (61) and the second layer (62) in the seat portion (22). The second layer (62) is higher in stiffness than the first layer (61) and the third layer (63). This feature can substantially prevent the second layer (62) from being downwardly dented together with the tube body (34). As a result, the internal pressure of the pressure sensitive tube (31) is significantly changed in response to the motion of the seated user (S), improving the detection accuracy of the pressure sensitive tube (31).

A third aspect of the present invention is an embodiment of the second aspect. In the third aspect, the seat portion (22) includes a fourth layer (64) arranged inward of the third layer (63) and having a higher stiffness than the third layer (63), and the fourth layer (64) has a notch (64a) that holds a portion of the pressure sensitive tube (31).

In the seat portion (22) of the third aspect, the fourth layer (64) is arranged inward of the third layer (63). The notch (64a) is formed in the fourth layer (64), and a portion of the pressure sensitive tube (31) is held in the notch (64a). Since the fourth layer (64) is higher in stiffness than the third layer (63), the pressure sensitive tube (31) can be held firmly in the notch (64a).

A fourth aspect of the present invention is an embodiment of any one of the first to third aspect. In the fourth aspect, the seat portion (22) has a surface inclined downwardly toward the backrest (23).

In the seat portion (22) of the fourth aspect, the surface of the seat portion (22) is inclined downwardly toward the backrest (23). Consequently, the seated user (S) is postured such that his/her buttocks are fitted in the space between the seat portion (22) and the backrest (23), thereby making it less easy for the seated user (S) to change his/her posture significantly. As a result, the motion of the seated user (S) can be detected with high accuracy.

A fifth aspect of the present invention is an embodiment of any one of the first to fourth aspect. In the fifth aspect, the biological information acquisition device further includes a leg rest (26) arranged in front of the seating article (21), and having a leg rest face (28) positioned higher than a front edge upper end of the seat portion (22).

In the fifth aspect, the leg rest (26) is arranged in front of the seating article (21). The leg rest face (28) of the leg rest (26) is located higher than the front edge upper end of the seating article (21). Consequently, in a state where the seated user (S) sits on the seating article (21) with his/her legs put on the leg rest (26), the seated user (S) is postured so as to sink back in the seating article (21) with his/her back leaning on the backrest (23). This feature can make it less easy for the seated user (S) to change his/her posture considerably, allowing the motion of the seated user (S) to be detected with high accuracy.

A sixth aspect of the preset invention is an embodiment of any one of the first to fifth aspects. In the sixth aspect, the pressure sensitive-part body (34) is arranged rearward of a middle portion of the seat portion (22) in a front-rear direction.

In the sixth aspect, the pressure sensitive-part body (34) is arranged rearward of the middle portion of the seat portion (22) in the front-rear direction. This feature makes it easy for the buttocks and the thighs of the seated user (S) to be positioned to correspond to the pressure sensitive-part body (34), allowing the motion of the seated user (S) to be detected with high accuracy.

### ADVANTAGES OF THE INVENTION

The present invention can ensure the comfort of the seated user (S), and enables the pressure sensitive part (31) to detect the motion with improved accuracy. Furthermore, according to the second aspect, the motion of the seated user (S) can be detected by using the pressure sensitive tube (31).

According to the third aspect, the pressure sensitive tube (31) can be held firmly in the notch (64a) of the fourth layer (64) so that displacement of the pressure sensitive-part body (34) can be substantially avoided. This enables the pressure sensitive part (31) to detect the motion with improved accuracy.

The fourth and fifth aspects can make it less easy for the seated user (S) to change his/her posture considerably, allowing the pressure sensitive part (31) to detect the motion with improved accuracy. According to the sixth aspect, the pressure sensitive-part body (34) is arranged close to the rear side of the seat portion (22), causing the motion of the seated user (S) to act reliably on the pressure sensitive part (31), and improving the accuracy of detection of the motion.

### BRIEF DESCRIPTION OF THE DRAWINGS

[FIG. 1] FIG. 1 is a schematic perspective view illustrating the overall configuration of a biological information acquisition device according to an embodiment.
[FIG. 2] FIG. 2 is a side view of the biological information acquisition device.
[FIG. 3] FIG. 3 is a longitudinal cross-sectional view illustrating the internal structure of a sofa.
[FIG. 4] FIG. 4 is a block diagram of the biological information acquisition device.
[FIG. 5] FIG. 5 is a cross-sectional view taken along line V-V in FIG. 3.
[FIG. 6] FIG. 6 is a view along an arrow VI in FIG. 5.
[FIG. 7] FIG. 7 is a cross-sectional view taken along line VII-VII in FIG. 5, and illustrating a state where no user is seated on the sofa.
[FIG. 8] FIG. 8 is a cross-sectional view taken along line VII-VII in FIG. 5, and illustrating a state where a user is seated on the sofa.
[FIG. 9] FIG. 9 is a longitudinal cross-sectional view illustrating the internal structure of a massage chair according to another embodiment.

### DESCRIPTION OF EMBODIMENTS

Embodiments of the present invention will be described in detail below, with reference to the drawings. Note that the embodiments described below are merely preferred examples in nature, and are not intended to limit the scope, application, or uses of the present invention.

### <Overall Configuration>

An embodiment of the present invention is configured as a biological information acquisition device (10) that acquires biological information of a human body. The biological information acquisition device (10) detects, as the biological information, a stress level or an autonomic nerve activity of a seated user (S).

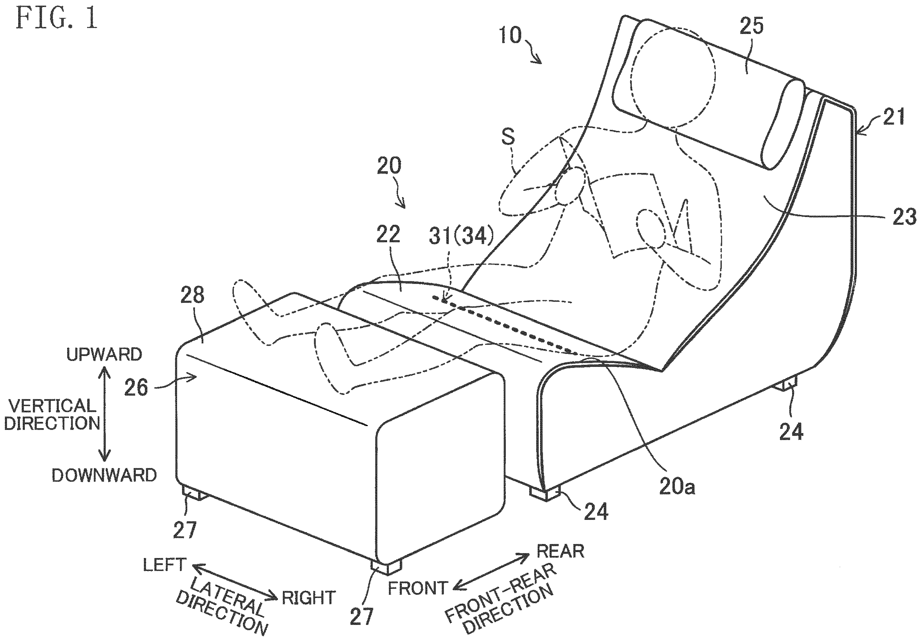

As illustrated in FIGS. 1, 2, and 3, the biological information acquisition device (10) includes a sofa set (20), an information acquisition unit (30), a signal output part (45), and a tablet terminal (50) (operation part).

### [Sofa Set]

The sofa set (20) includes a single-seat sofa (21) (seating article), and an ottoman (26) (leg rest) arranged in front of the sofa (21). The surface of the body of each of the sofa (21) and the ottoman (26) is covered with synthetic leather (20a) (e.g., polyurethane-made leather).
Note that any other covering material, such as natural leather or a fabric, may be used to cover the surface of the body of each of the sofa (21) and the ottoman (26).

The sofa (21) includes a seat portion (22), a backrest (23) formed on the rear side of the seat portion (22), and four legs (24) that support the sofa (21).

The seat portion (22) has a surface inclined downwardly toward the rear side (backrest (23)). The seat portion (22) is located to correspond to the buttocks and the thighs of the seated user (S). The backrest (23) is located to correspond to the back and the head of the seated user (S). The backrest (23) has a surface upwardly inclined toward the rear side. That is, in the sofa (21), the seat portion (22) and the backrest (23) form a substantial V-shape as viewed from side. The backrest (23) has a cushion (25) attached to an upper portion thereof, the upper portion corresponding to the head of the seated user (S).

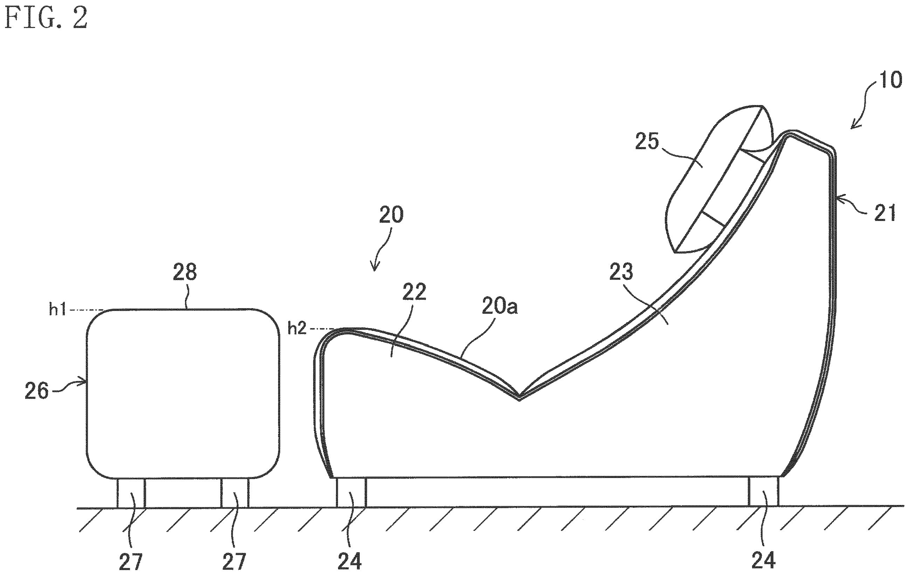

The ottoman (26) is formed in a rectangular parallelepiped shape with an upper surface having a substantially square shape, and supported by four legs (27). The upper surface of the ottoman (26) is formed as a leg rest face (28) on which the legs of the seated user (S) are placed. As illustrated in FIG. 2, a height h1 of the leg rest face (28) of the ottoman (26) is greater than a height h2 of a front edge upper end of the seat portion (22).

### [Biological Information Acquisition Unit]

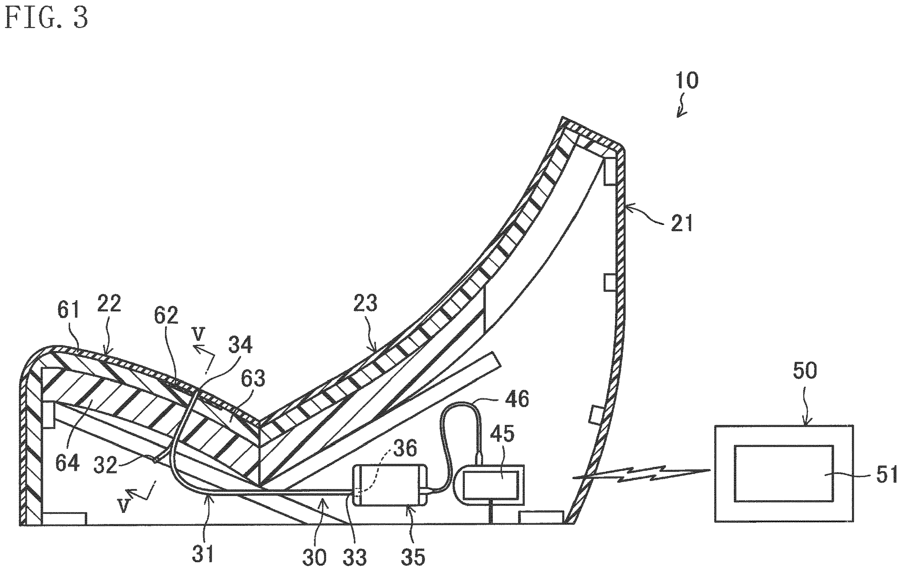

As illustrated in FIG. 3, the information acquisition unit (30) includes a pressure sensitive tube (31), and a sensor body (35).

The pressure sensitive tube (31) constitutes a pressure sensitive part that receives pressure caused by a motion of the seated user (S). The pressure sensitive tube (31) is comprised of a tube whose inside diameter is approximately 4 mm, the tube being made of a resin (e.g., vinyl chloride). One end of the pressure sensitive tube (31) is closed by a sealing part (32). The other end of the pressure sensitive tube (31) is connected to the sensor body (35) via a connecting portion (33). An intermediate portion of the pressure sensitive tube (31) constitutes a pressure sensitive-part body (tube body (34)) arranged in the vicinity of the surface of the seat portion (22) of the sofa (21). The tube body (34) and its peripheral structure will be described in detail later.

Note that the pressure sensitive tube (31) may be configured such that the communication between the inside and the outside of the pressure sensitive tube (31) is established to such an extent that the internal pressure of the pressure sensitive tube (31) varies in response to a motion of the seated user (S). In this case, for example, the one end of the pressure sensitive tube (31) may be opened, or a throttle member having an opening may be attached to the one end of the pressure sensitive tube (31).

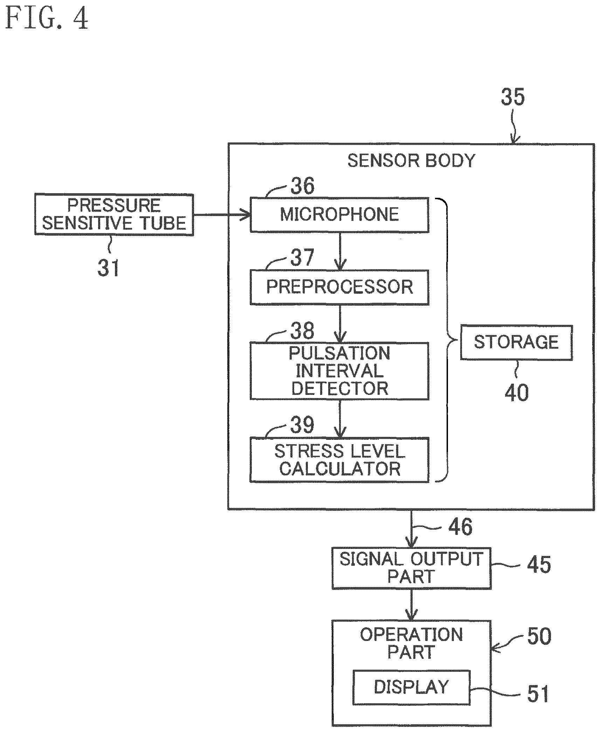

As illustrated in FIG. 3, the sensor body (35) is arranged inside the sofa (21). As illustrated in FIG. 4, the sensor body (35) includes a microphone (36) that detects the internal pressure of the pressure sensitive tube (31). The microphone (36) receives the internal pressure of the pressure sensitive tube (31) to output a pressure signal.

As illustrated in FIG. 4, the sensor body (35) includes a preprocessor (37), a pulsation interval detector (38), a stress level calculator (39), and storage (40).

The preprocessor (37) performs predetermined filtering processing on the signal output from the microphone (36). The pulsation interval detector (38) derives an R wave, which is large in amplitude, from the signal having been subjected to the filtering processing. The pulsation interval detector (38) then calculates the length of a pulsation interval for each predetermined section.

In order to perform the frequency analysis of the variation of the pulsation interval (fluctuation of the pulsation interval) calculated, the stress level calculator (39) converts pulsation interval data into isochronous interval data by linear interpolation. Thereafter, the fast Fourier transform (FFT) is performed with respect to the pulsation interval data that is isochronous interval data, thereby obtaining the ratio (LH/HF) of a low frequency component LF (e.g., from 0.04 Hz to 0.15 Hz) of the fluctuation of the pulsation interval to a high frequency component HF (e.g., 0.15 Hz or higher). The ratio (LH/HF) constitutes biological information related to the stress level or the autonomic nerve activity. For example, when the ratio (LH/HF) is not less than a first predetermined value ("2", for example), it is possible to determined that the stress level is high, and when the ratio (LH/HF) is not less than a second predetermined value ("5", for example), it is possible to determine that the seated user (S) is in an excessively stressful state.

The storage (40) timely stores, for example, the pressure signal detected by the microphone (36), the pulsation interval detected by the pulsation interval detector (38), the stress level calculated by the stress level calculator (39). Note that the storage (40) may store only the pressure signal, and calculate the other indexes later when necessary.

### [Signal Output Part and Operation Part]

As illustrated in FIGS. 3 and 4, the signal output part (45) is connected to the sensor body (35) of the information acquisition unit (30) through a cable (e.g., a USB cable (46)). The signal output part (45) wirelessly outputs the signals (e.g., the pressure signal, the pulsation interval, and the stress level) that have been output from the sensor body (35) to the tablet terminal (50). Note that the signal output part (45) may be configured to output the predetermined signals not wirelessly but through a wire. The tablet terminal (50) has a display (51) configured to display the stress level and the like that are calculated by the sensor body (35).

### [Tube Body and Peripheral Structure]

The tube body (34) and its peripheral structure are now described in detail with reference to FIG. 3, and FIGS. 5 to 8. The tube body (34) is, as illustrated in FIG. 3, arranged in the vicinity of the surface of the seat portion (22) of the sofa (21). The tube body (34) extends straight in the width direction (lateral direction) of the seat portion (22). The tube body (34) is located slightly rearward of a middle portion of the seat portion (22) in the front-rear direction.

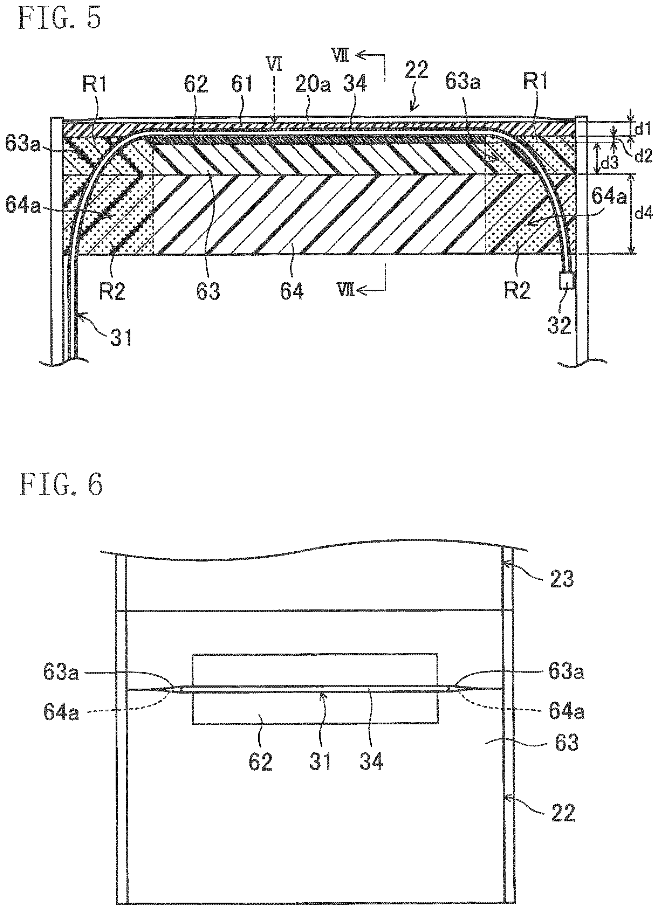

As illustrated in FIG. 5, the seat portion (22) is comprised of a plurality of layers (members) stacked on top each other. Specifically, the seat portion (22) is comprised of synthetic leather (20a), resin cotton (61) (first layer), PVC leather (62) (polyvinyl chloride leather (second layer)), urethane (63) (third layer), and chip urethane (64) (fourth layer) that are stacked sequentially from the top side to the bottom side of the seat portion (22).

The layer of resin cotton (61) has a thickness d1, the PVC leather (62) has a thickness d2, the layer of urethane (63) has a thickness d3, and the layer of chip urethane (64) has a thickness d4. In the seat portion (22), the relation expressed as d2<d1<d3<d4 is satisfied. Note that these thicknesses may satisfy the relation expressed as dl=d3 or dl>d3.

The resin cotton (61) has a stiffness S1, the PVC leather (62) has a stiffness S2, the urethane (63) has a stiffness S3, the chip urethane (64) has a stiffness S4, and the pressure sensitive tube has a stiffness S5. In the seat portion (22), the relation expressed as S1<S3<S2≈ S5<S4 is satisfied.

As illustrated in FIGS. 5 and 6, the tube body (34) is sandwiched and held between the PVC leather (62) and the resin cotton (61). The layer of PVC leather (62) has a width and a length that are sufficient to cover the tube body (34) from below (see FIG. 6).

The layer of urethane (63) has, in each of its both end portions in the width direction, an upper-side notch (63a). Each of the upper-side notches (63a) extends from the right or left side of the layer of urethane (63) in the width direction to reach an associated one of both end portions of the layer of PVC leather (62). The layer of chip urethane (64) has, in each of its both end portions, a lower-side notch (64a). Each of the lower-side notches (64a) extends from the right or left side of the layer of chip urethane (64) in the width direction to a location corresponding to an associated one the both end portions of the layer of PVC leather (62). That is, the lower-side notches (64a) are located to correspond to the upper-side notches (63a) in the vertical direction. The upper-side notches (63a) are formed in regions R1 indicated with dot hatching in FIG. 5, and the lower-side notches (64a) are formed in regions R2 indicated with dot hatching in FIG. 5.

A portion, of the pressure sensitive tube (31), which extends from the tube body (34) toward the sealing part (32) is held in the notch (63a) and the notch (64a) located in one end portion of the seat portion (22) in the width direction. A portion, of the pressure sensitive tube (31), which extends from the tube body (34) toward the connecting portion (33) is held in the notch (63a) and the notch (64a) located in the other end portion of the seat portion (22) in the width direction. Consequently, the pressure sensitive tube (31) is reliably fixed to the seat portion (22), substantially preventing displacement of the tube body (34) in the seat portion (22).

### - Operation of Seat Portion and Other Components -

In this embodiment, the tube body (34) is entirely covered with the resin cotton (61). The resin cotton (61) is lower in stiffness than the pressure sensitive tube (31), and has flexibility. Therefore, the pressure sensitive tube (31) is less likely to be perceived by the buttocks and the thighs of the user (S) seated on the seat portion (22), and the seated user (S) is substantially prevented from feeling uncomfortable on the seat portion (22). Accordingly, it is possible to ensure the comfort of the seated user (S).

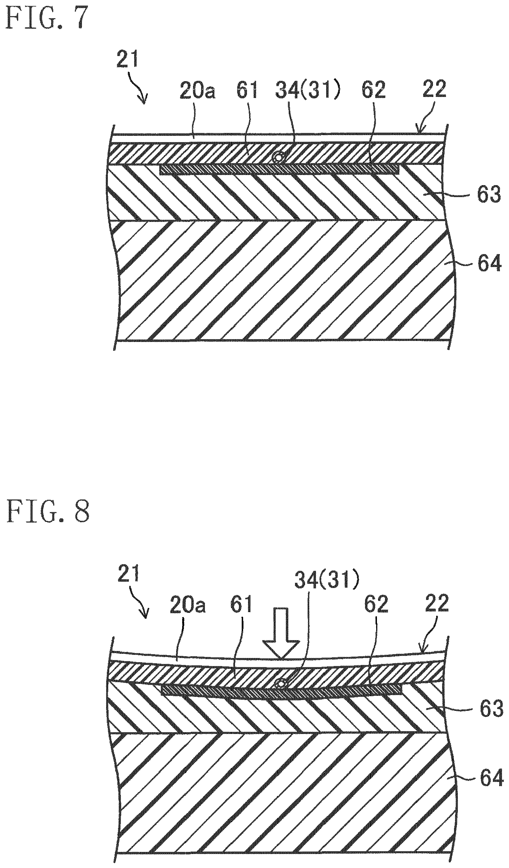

The tube body (34) is arranged on the upper surface of the PVC leather (62). The PVC leather (62) is higher in stiffness than the urethane (63) arranged below the PVC leather (62). As can be seen from FIGS. 7 and 8, this feature can substantially prevent the tube body (34) from being significantly depressed together with the urethane (63) when a motion of the seated user (S) (indicated by the hollow arrow in FIG. 8) acts on the seat portion (22). That is, the PVC leather (62) is capable of preventing the tube body (34) from being downwardly displaced and hence, the internal pressure of the tube body (34) is significantly changed, contributing to improvement of the accuracy of the pressure signal.

The pressure sensitive tube (31) is held in the lower-side notches (64a, 64a) of the chip urethane (64) having a comparatively high stiffness. Due to this configuration, displacement of the pressure sensitive tube (31) can be substantially prevented, resulting in substantial prevention of the displacement of the tube body (34) in the seat portion (22). As a result, it is possible to prevent the accuracy of the pressure signal from being deteriorated due to the displacement of the tube body (34).

The surface of the seat portion (22) is inclined downward toward the backrest (23). Therefore, the user (S) seated on the sofa (21) is postured such that his/her buttocks are fitted in the space between the seat portion (22) and the backrest (23), making it less easy for the seated user (S) to change the posture. This feature can substantially prevent the buttocks and the thighs of the seated user (S) from being deviated from the tube body (34), contributing to substantially prevention of deterioration of the accuracy of the pressure signal.

In the sofa set (20), the height h1 of the leg rest face (28) of the ottoman (26) is greater than the height h2 of the front edge upper end of the sofa (21) (see FIG. 2). Accordingly, in a state where the seated user (S) lies with his/her legs put on the leg rest face (28) of the ottoman (26) (see FIG. 1), the seated user (S) is postured such that his/her buttocks are fitted in the space between the seat portion (22) and the backrest (23), making it less easy for the seated user (S) to change the posture. This feature can keep more reliably the buttocks and the thighs of the seated user (S) from being deviated from the tube body (34), contributing to substantially prevention of deterioration of the accuracy of the pressure signal.

In the seat portion (22), the tube body (34) is arranged slightly rearward of the middle portion of the seat portion (22) in the front-rear direction. Thanks to this feature, the buttocks and the thighs of the seated user (S) are positioned to correspond to the tube body (34), contributing to further improvement of the accuracy of the pressure signal.

### - Advantageous Effects of Embodiment -

According to the embodiment described above, the tube body (34) is sandwiched between the resin cotton (61) having flexibility and the PVC leather (62) having a certain stiffness (flexural rigidity). This feature can ensure the comfort of the seated user (S), and allow the pressure sensitive tube (31) to detect a motion with improved accuracy.

Furthermore, as illustrated in FIG. 6, it is possible firmly to hold the pressure sensitive tube (31) in the notches (64a) of the chip urethane (64), and to avoid displacement of the pressure sensitive tube (31). This consequently allows the pressure sensitive tube (31) to detect a motion with improved accuracy.

The embodiment described above makes it less easy for the seated user (S) to change his/her posture, allowing the pressure sensitive tube (31) to detect a motion with improved accuracy. Furthermore, the tube body (34) is arranged closer to the rear side of the seat portion (22). This configuration enables a motion of the seated user (S) to act reliably on the pressure sensitive tube (31), contributing improvement of the accuracy of detection of the motion.

### ≪ Other embodiments ≫

In the embodiment described above, the pressure sensitive tube (31) is used as a pressure sensitive part, and the tube body (34) of the pressure sensitive tube (31) is arranged between the first layer (61) and the second layer (62). Alternatively, another component, such as a piezoelectric sheet that receives pressure caused by a motion of the seated user (S), may be used as the pressure sensitive part, and a portion of the piezoelectric sheet may be arranged between the first layer (61) and the second layer (62) so as to function as a body of the pressure sensitive part.

Another member, such as a sheet-like member or a plate-like member, may be stacked between the first layer (61) and the pressure sensitive tube (31). Examples of this member includes a pressure receiving plate used for increasing a pressure receiving area for receiving pressure from the seated user (S).

Furthermore, in the embodiment described above, the seating article (sofa (21)) and the leg rest (ottoman (26)) are formed separately from each other. However, the present invention may be applied to a seating article which is long in the front-rear direction, the chair corresponding to the seating article (21) with which the leg rest (26) is integrated. Alternatively, the ottoman (26) may be omitted from the biological information acquisition device (10).

Each of the respective materials for the first layer (61), the second layer (62), the third layer (63), and the fourth layer (64) that together form the seat portion (22) is merely an example. That is, any other material may be used for each of these layers, provided that the stiffness of the respective layers satisfies the relation mentioned above. Specifically, as a combination of the materials different from that of the embodiment described above, for example, the first layer (61) may be made of leather, the second layer (62) may be made of urethane, the third layer (63) may be made of chip urethane, and the fourth layer (64) may be made of any one of polyethylene, polypropylene, vinyl chloride, PET (polyethylene terephthalate), and an acrylate resin.

In the embodiment described above, the synthetic leather (20a) is stacked on the outside of the first layer (61). Alternatively, the synthetic leather (20a) may be omitted. That is, the seating article (21) may include the first layer (61) as the outermost surface of the seat portion (22).

Although the seat portion (22) according to the present invention is applied to the sofa (21), the seat portion (22) is usable for any object provided that the object allows a person to sit thereon. For example, the seat portion (22) is usable for a seat (including a passenger seat or a driver seat) of transport machinery (e.g., an automotive, an airplane, and an electric train), or for a massage chair.

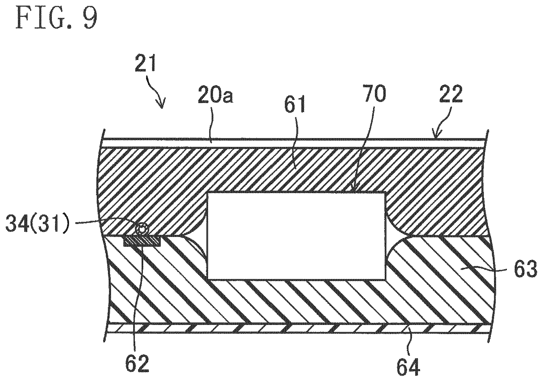

FIG. 9 illustrates an example in which the present invention is applied to the seat portion (22) of a massage chair. The seat portion (22) includes a first layer (61), a second layer (62), a third layer (63), and a fourth layer (64) that are stacked sequentially below the cover (20a). The first layer (61) is comprised of resin cotton and has a comparatively large thickness, and the second layer (62) is comprised of PVC leather having a comparatively small thickness. The third layer (63) is comprised of urethane and has a comparatively large thickness, and the fourth layer (64) is comprised of a support base having a comparatively small thickness and high stiffness.

A vibration body (70) having a vibrator is interposed between the first layer (61) and the third layer (63). When the vibration body (70) vibrates, the vibration of the vibration body (70) is transmitted to the seated user (S) through the first layer (61). A tube body (34) of a pressure sensitive tube (31) is interposed between the first layer (61) and the second layer (62). Also in this example, the tube body (34) is sandwiched between the first layer (61) having flexibility and the PVC leather (62) having a comparatively high stiffness. This configuration can ensure the comfort of the seated user (S), and allows the pressure sensitive tube (31) to detect a motion with improved accuracy.

### INDUSTRIAL APPLICABILITY

As described above, the present invention is useful for a biological information acquisition device.

### DESCRIPTION OF REFERENCE CHARACTERS

- 10: Biological Information Acquisition Device
- 21: Sofa (Seating Article)
- 22: Seat Portion
- 23: Backrest
- 26: Leg Rest
- 28: Leg Rest Face
- 30: Information Acquisition Unit (Information Acquisition Part)
- 31: Pressure Sensitive Tube (Pressure Sensitive Part)
- 34: Tube Body (Pressure Sensitive-Part Body)
- 61: Resin Cotton (First Layer)
- 62: PVC Leather (Second Layer)
- 63: Urethane (Third Layer)
- 64: Chip Urethane (Fourth Layer)
- 64a: Lower-Side Notch (Notch)
- S: Seated User

## Claims

1. A biological information acquisition device comprising:
a seating article (21) having a backrest (23) and a seat portion (22);
a pressure sensitive part (31) arranged in the seat portion (22), and configured to receive pressure caused by a motion of a seated user (S); and
an information acquisition part (30) configured to acquire biological information of the seated user (S) based on the pressure received by the pressure sensitive part (31), wherein
the seat portion (22) includes a first layer (61), a second layer (62), and a third layer (63) that are stacked sequentially from a surface side to an inner side of the seat portion (22), the second layer (62) has a higher stiffness than the third layer (63),
the first layer (61) has a lower stiffness than the third layer (63), and
the pressure sensitive part (31) has a pressure sensitive-part body (34) arranged between the first layer (61) and the second layer (62).

2. The biological information acquisition device of claim 1, wherein
the pressure sensitive part includes a pressure sensitive tube (31) whose internal pressure changes in response to the motion of the seated user (S), and
the pressure sensitive-part body includes a tube body (34) arranged between the first layer (61) and the second layer (62).

3. The biological information acquisition device of claim 2, wherein
the seat portion (22) includes a fourth layer (64) arranged inward of the third layer (63) and having a higher stiffness than the third layer (63), and
the fourth layer (64) has a notch (64a) that holds a portion of the pressure sensitive tube (31).

4. The biological information acquisition device of any one of claims 1 to 3, wherein
the seat portion (22) has a surface inclined downwardly toward the backrest (23).

5. The biological information acquisition device of any one of claims 1 to 4, further comprising:
a leg rest (26) arranged in front of the seating article (21), and having a leg rest face (28) positioned higher than a front edge upper end of the seat portion (22).

6. The biological information acquisition device of any one of claims 1 to 5, wherein
the pressure sensitive-part body (34) is arranged rearward of a middle portion of the seat portion (22) in a front-rear direction.
